# DEMANDE DE BREVET EUROPEEN

(11) **EP 1 695 688 A1**
(43) Date de publication de la demande: **30.08.2006**
(21) Numéro de dépôt: 06290315.8
(22) Date de dépôt: 24.02.2006
(51) Int. Cl.: A61K 8/02, A61K 8/49, A61Q 5/10

(54) **Composition anhydre sous forme de film comprenant un polymère filmogène et un colorant direct**

(30) Priorité: 28.02.2005 FR 0502032
(71) Demandeur: L'ORÉAL, 75008 Paris (FR)
(72) Inventeur: Bone, Eric, Setagaya-ku 158-0091 Tokyo (JP); Cotteret, Jean, 78480 Verneuil sur Seine (FR)
(74) Mandataire: Fevrier, Murielle Françoise E.

(57) **Abrégé**

La présente invention a pour objet une composition anhydre sous forme de film comprenant au moins un polymère filmogène et au moins un colorant direct, la teneur totale en colorant direct étant d'au moins 3 % en poids par rapport au poids de la composition

Elle a de plus pour objet un procédé de préparation d'une telle composition dans lequel on applique sur un support, une composition précurseur comprenant, dans un solvant approprié, un mélange comprenant au moins un colorant direct, au moins un polymère filmogène ; puis on évapore ledit solvant.

Elle concerne enfin un procédé de coloration de fibres kératiniques, consistant à mettre en contact lesdites fibres et la composition, en présence d'un milieu aqueux.

## Description

La présente invention a pour objet une composition anhydre sous forme de film comprenant au moins un polymère filmogène et au moins un colorant direct, ainsi qu'un procédé préparation de ladite composition. Elle concerne de plus un procédé de coloration de fibres kératiniques, notamment humaines, mettant en oeuvre une telle composition.

Il est connu depuis longtemps de modifier la couleur des cheveux, et en particulier de masquer les cheveux blancs.

On connaît essentiellement deux types de technologies mises en oeuvre pour la coloration des fibres kératiniques humaines.

La première méthode, appelée coloration directe ou semi-permanente, consiste à changer ou apporter de la couleur par l'application d'une molécule colorée qui pénètre par diffusion à l'intérieur de la fibre et/ou reste adsorbée à sa surface. On peut aussi mettre en oeuvre cette coloration en présence d'un agent alcalin en conditions oxydantes. Dans un tel cas, on observe à la fois un éclaircissement des fibres accompagné d'une coloration de ces dernières. On parle alors de coloration directe en conditions éclaircissantes.

La seconde méthode, appelée coloration d'oxydation ou coloration permanente, consiste à changer ou apporter de la couleur en mettant en oeuvre à l'intérieur même de la fibre, une condensation oxydative de précurseurs de colorants qui sont des composés peu ou non colorés. Après cette réaction, les colorants formés sont insolubles et sont piégés à l'intérieur de la fibre.

Les deux méthodes résumées ci-dessus permettent d'accéder à de nombreuses couleurs, puissantes, assez tenaces et peu sélectives.

L'invention se rapporte plus particulièrement au domaine de la coloration directe.

L'un des inconvénients parfois rencontrés réside dans le fait ces colorants peuvent être fragiles, voire instables en solution aqueuse diluée, que ces derniers soit ou non en présence d'agents alcalins.

En outre, de nombreux colorants directs sont instables en présence d'agents réducteurs ou oxydants.

Pour pallier ces inconvénients, on propose parfois de les présenter dans un conditionnement spécifique, comme par exemple des sachets pour poudres, ou encore de les associer dans la composition à des agents de stabilisation efficaces.

On peut aussi rencontrer d'autres difficultés dues au fait que très fréquemment, les compositions tinctoriales sont mélangées extemporanément avant l'application, à des compositions annexes. Ainsi, de tels modes de réalisation conduisent à des mises en oeuvre longues et parfois difficiles tout en ne garantissant pas une homogénéité parfaite de la composition à appliquer sur les fibres.

La présente invention a pour but de résoudre les inconvénients mentionnés ci-dessus.

Un premier objet de l'invention est donc constitué par une composition sous forme de film comprenant au moins un polymère filmogène et au moins un colorant direct, la teneur totale en colorant direct étant d'au moins 3 % en poids par rapport au poids de la composition.

Elle a de même pour objet un procédé de préparation d'une telle composition dans lequel on applique sur un support, une composition précurseur comprenant, dans un solvant approprié, un mélange comprenant au moins un colorant direct, au moins un polymère filmogène ; puis on évapore ledit solvant

Elle a enfin pour objet un procédé de coloration de fibres kératiniques, notamment humaines, consistant à mettre en contact lesdites fibres et la composition, en présence d'un milieu aqueux.

La composition permet de résoudre le problème de stabilité des colorants directs durant le stockage, sans avoir nécessairement recours à l'emploi d'agents de stabilisation. En effet, les colorants directs sont protégés par le film de polymère.

A noter par ailleurs que les conditions d'application de la composition sont simplifiées. En effet, on peut s'affranchir de l'étape de mélange de la composition tinctoriale et de la composition oxydante avant l'application sur les fibres. On peut aussi procéder à un tel mélange mais la nature de la composition selon l'invention est telle que le mélange et l'homogénéisation des compositions sont considérablement simplifiés.

Mais d'autres caractéristiques de la présente invention apparaîtront plus clairement à la lecture de la description et des exemples qui vont suivre.

Dans ce qui va suivre, et à moins qu'une indication différente ne soit donnée, lorsqu'une gamme de valeurs est donnée, les bornes de cette gamme sont incluses dans le domaine ainsi défini.

La présente invention s'applique au traitement des fibres kératiniques, plus particulièrement humaines, et notamment les cheveux.

Au sens de l'invention, la composition est considérée anhydre car sa teneur en eau est inférieure à 10 % en poids de la composition, plus particulièrement inférieure à 5 % en poids, de préférence inférieure à 3 % en poids, par rapport au poids de la composition. De préférence, la composition ne contient pas d'eau.

Comme cela a été indiqué plus haut, la composition anhydre selon l'invention se trouve sous la forme d'un film comprenant au moins un polymère filmogène et au moins un colorant direct, la teneur totale en colorant direct étant d'au moins 3 % en poids par rapport au poids de la composition.

Le ou les colorants directs entrant dans la composition selon l'invention sont avantageusement choisis parmi les colorants directs classiquement utilisés dans le domaine de la coloration des fibres kératiniques, et notamment des fibres kératiniques humaines.

Ainsi, ces colorants directs peuvent être des espèces ioniques ou non ioniques. De préférence, lesdits colorants directs sont choisis parmi des espèces cationiques ou non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthytoxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthy))amino-4-N,N-bis-(β-hydroxyéthy))amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 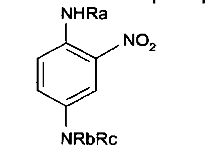
dans laquelle :
- Rb représente un radical alkyle en C₁-C₄, un radical β-hydroxyéthyle ou β-hydroxypropyle ou γ-hydroxypropyle ;
- Ra et Rc, identiques ou différents, représentent un radical β-hydroxyéthyle -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17, Basic Red 2.

Parmi les colorants directs méthiniques cationiques, on peut aussi citer le Basic Red 14, le Basic Yellow 13 et le Basic Yellow 29.

Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-chloro-4'-hydroxy)phényl-acétylamino-6-méthoxy-1,4-benzoquinone imine
- 3-N(3'-chloro-4'-méthylamino)phényl-uréido-6-méthyl-1,4-benzoquinone imine
- 3-[4'-N-(éthylcarbamylméthyl)-amino]-phényl-uréido-6-méthyl-1,4-benzoquinone imine.

La composition peut aussi comprendre des colorants directs naturels comme la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine.

Selon un mode de réalisation particulier de l'invention, la teneur totale en colorant direct est comprise entre 3 et 99,5 % en poids par rapport au poids de la composition.

De préférence, la teneur totale en colorant direct est comprise entre 10 et 80 % en poids par rapport au poids de la composition.

La composition selon l'invention comprend en outre au moins un polymère filmogène, qui peut avantageusement être non ionique, cationique, anionique ou amphotère.

Il est à noter que le polymère filmogène entrant dans la composition selon l'invention est tel que dans les conditions d'application de la composition sur les fibres kératiniques, c'est-à-dire notamment en présence d'un milieu aqueux et de préférence, d'un massage des fibres ou d'une homogénéisation, la composition perd sa cohésion et se désagrège.

Plus particulièrement, le polymère filmogène est choisi parmi les polymères dérivés de vinylpyrolidone, l'alcool polyvinylique, les polyuréthanes, les polymères dérivés de caprolactame, vinyllactame, acétate de vinyle, les polymères dérivés d'acrylamide, les polysaccharides capables de former un film à l'état sec tels que les dérivés cellulosiques, les amidons et dérivés, la gomme pullulane, la gomme arabique, les pectines, les alginates, les carraghénanes, les galactomannanes, les agars, les chitosans, les chitines, les polymères dérivés d'acide hyaluronique, gomme de xanthane, gomme de karaya, les protéines capables de former un film à l'état sec tels que la gélatine, le gluten, la caséine, la zéine, la gliadine, l'hordéine et leurs dérivés naturels ou synthétiques, les polymères dérivés de silicones, les polymères amphotères ou anioniques dérivant de monomères comprenant au moins une fonction carboxylique, sulfonique ou phosphorique, les copolymères acrylique de phosphoryle choline (lipidure), les complexes anion-cation type gomme arabique / gélatine ou gomme arabique / chitosane, ou l'association collagène / GlycosAminoGlycane.

A titre de polymères filmogènes cationiques convenables, on peut citer plus particulièrement les polymères suivants, ayant en général une masse moléculaire moyenne en nombre comprise entre 500 et environ 5 000 000 :
(1) les homopolymères ou copolymères dérivés d'esters ou d'amides acryliques ou méthacryliques et comportant au moins un des motifs de formules suivantes : 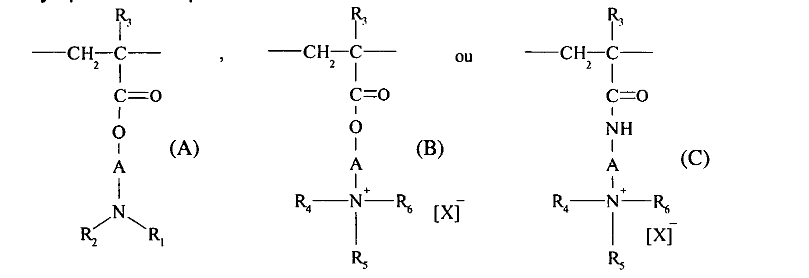 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
(2) les gommes de guar quaternisées ;
(3) les copolymères quaternaires de vinylpyrrolidone et de vinylimidazole ;
**(4)** les chitosanes ou leurs sels ;
   les sels utilisables sont en particulier l'acétate, le lactate, le glutamate, le gluconate ou le pyrrolidone-carboxylate de chitosane.

Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques
ou méthacryliques ou de leurs esters, de vinyllactames tels que la vinylpyrrolidone ou le vinylcaprolactame, d'esters vinyliques.

Ainsi, parmi ces copolymères de la famille (1), on peut citer :
- les copolymères d'acrylamide et de méthacrylate de diméthyl-amino-éthyle quaternisés au sulfate de diméthyle ou avec un halogénure de diméthyle,
- les copolymères d'acrylamide et de chlorure de méthacryloyloxy-éthyl-triméthyl-ammonium décrits, par exemple, dans la demande de brevet EP-A-080976,
- les copolymères d'acrylamide et de méthosulfate de méthacryloyloxy-éthyl-triméthyl-ammonium,
- les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français 2 077 143 et 2 393 573,
- les terpolymères méthacrylate de diméthylaminoéthyle / vinylcaprolactame / vinylpyrrolidone tels que le produit commercialisé sous la dénomination GAFFIX® VC 713 par la société ISP, et
- les copolymères vinylpyrrolidone/méthacrylamide de diméthyl-amino-propyle quaternisé tels que notamment le produit commercialisé sous la dénomination "GAFQUAT® HS 100" par la société ISP.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

En ce qui concerne les polymères filmogènes anioniques, ces derniers comprennent généralement au moins un groupement dérivé d'acide carboxylique, sulfonique ou phosphorique et ont une masse moléculaire moyenne en nombre comprise entre environ 500 et 5 000 000.

Les groupements carboxyliques sont plus particulièrement apportés par des monomères mono ou diacides carboxyliques insaturés tels que ceux répondant à la formule : 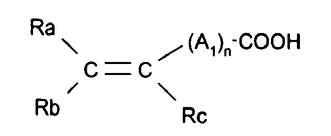 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, Ra désigne un atome d'hydrogène, un groupement phényle ou benzyle, Rb désigne un atome d'hydrogène, un groupement alkyle en C1-C4, en particulier méthyle, éthyle, ou carboxyle, Rc désigne un atome d'hydrogène, un groupement alkyle inférieur, un groupement -CH₂-COOH, phényle ou benzyle ;

Les polymères filmogènes anioniques à groupements carboxyliques préférés sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacryliquelacrylate d'éthylelacrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques mono-insaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, alpha-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates ;
F) les polyuréthannes anioniques, tels que le produit commercialisé par BASF sous la dénomination Luviset PUR.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, on peut également utiliser les polymères anioniques filmogènes de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 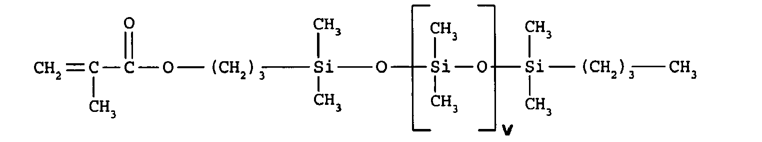
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères filmogènes, des polyuréthannes fonctionnalisés, siliconés ou non.

Les polyuréthannes particulièrement visés par la présente invention sont ceux décrits dans les brevets EP 0 751 162, EP 0 637 600, FR 2 743 297 et EP 0 648 485, ainsi que le brevets EP 0 656 021 ou WO 94/03510 et EP 0 619 111.

Selon l'invention, les polymères filmogènes anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF, les copolymères acétate de vinyle/acide crotonique et les copolymères acétate de vinyle/acide crotonique greffés par du polyéthylèneglycol vendus sous la dénomination ARISTOFLEX® A par la société BASF ainsi que le polyuréthanne Luviset PUR® commercialisé par la Société BASF.

Les polymères filmogènes anioniques les plus particulièrement préférés sont choisis parmi les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP ainsi que le polyuréthanne Luviset PUR® commercialisé par la Société BASF.

Parmi les polymères filmogènes amphotères utilisables on peut citer ceux comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-alpha-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique (de préférence une fonction amino) tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diéthyldiallylammonium. Les copolymères d'acide acrylique et de ce dernier monomère sont proposés sous les appelations MERQUAT 280, MERQUAT 295 et MERQUAT PLUS 3330 par la société CALGON.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthyl-amino-éthyle avec le sulfate de diméthyle ou diéthyle.
   Les acrylamides ou méthacrylamides N-substitués plus particulièrement préférés sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'amino-éthyle, de butylaminoéthyle, de N,N'-diméthyl-aminoéthyle, de N-tertio-butyl-aminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale : 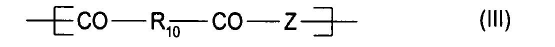 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 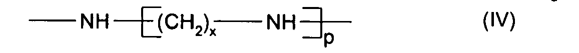 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 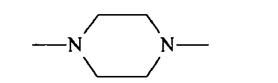
   c) dans les proportions de 0 à 20 % en moles, le groupe -NH-(CH₂)₆-NH- dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
      Les alcane-sultones utilisées dans l'acylation sont de préférence la propane- ou la butane-sultone, les sels des agents d'acylation sont de préférence les sels de sodium ou de potassium.
**(4)** les polymères comportant des motifs zwittérioniques de formule : 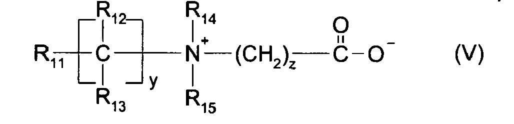 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle
   ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthyl-aminoéthyle ou des acrylates ou méthacrylates d'alkyle, des acrylamides ou méthacrylamides, ou l'acétate de vinyle.
   A titre d'exemple, on peut citer les copolymères méthacrylate de butyle/méthacrylate de N,N-diméthylcarboxy-aminoéthyl.
(5) les polymères dérivés du chitosane comportant des motifs monomères répondant aux formules suivantes : 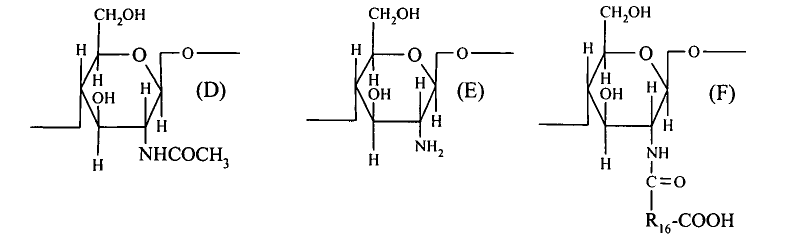 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 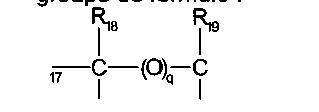 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (VI) sont, par exemple, décrits dans le brevet français 1 400 366 et comprenant le motif de répétition ci-dessous : 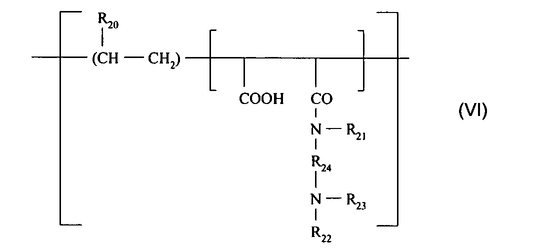 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane.
(8) Les polymères amphotères du type -D-X-D-X choisis parmi:
   a) les polymères obtenus par action de l'acide chloracétique ou le chloracétate de sodium sur les composés comportant au moins un motif de formule: -D-X-D-X-D- (VII)
      où D désigne un groupe 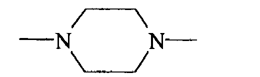 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule : -D-X-D-X- (VII')
      où D désigne un groupe 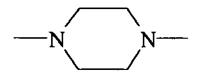 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** les copolymères alkyl(C₁-C₅)-vinyléther/anhydride maléique modifiés partiellement par semiamidification avec une N,N-dialkylaminoalkylamine telle que la N,N-diméthyl-amino-propylamine ou par semiestérification avec un N,N-dialkylaminoalcanol. Ces copolymères peuvent également comporter d'autres comonomères vinyliques tels que le vinylcaprolactame.

Les polymères filmogènes amphotères préférés sont ceux de la famille (3) tels que les copolymères dont la dénomination CTFA est Octylacrylamide/acrylates/butylaminoethylmethacrylate copolymer, tels que les produits vendus sous les dénominations AMPHOMER® , AMPHOMER® LV 71 ou LOVOCRYL® 47 par la société NATIONAL STARCH et ceux de la famille (4) tels que les copolymères méthacrylate de butyle/méthacrylate de N,N-diméthylcarboxyaminoéthyle.

Les polymères filmogènes non ioniques utilisables selon la présente invention sont choisis, par exemple, parmi :
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHODIA CHIMIE ;
- les copolymères de styrène, de méthacrylate d'alkyle et d'acrylate d'alkyle
- les polyuréthannes non ioniques,
- les copolymères de styrène et de butadiène ;
- les copolymères de styrène, de butadiène et de vinylpyridine;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères filmogènes sont de préférence des polymères non ioniques, et mieux encore des polymères non ioniques à motifs vinyllactames. Ils sont notamment décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.

Les homopolymères ou copolymères à motifs vinyllactame comprennent des motifs de formule : 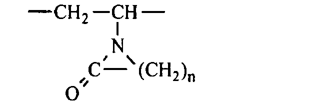 dans laquelle n est indépendamment 3, 4 ou 5.

La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

On peut utiliser, en particulier, comme polymère filmogène, dans la présente invention, les polyvinylpyrrolidones telles que celles commercialisées sous la dénomination Luviskol® K30 par la société BASF ; les polyvinylcaprolactames tels que ceux commercialisés sous la dénomination Luviskol® PLUS par la société BASF ; les copolymères poly(vinylpyrrolidone/acétate de vinyle) tels que ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol® VAP 343 par la société BASF.

Les polymères filmogènes entrant dans la composition selon l'invention sont plus particulièrement choisis parmi les polymères filmogènes solubles ou hydrodispersables. De préférence, les polymères filmogènes sont choisis parmi les polymères filmogènes hydrosolubles.

La teneur en polymère filmogène représente avantageusement de 0,5 à 97 % en poids par rapport au poids de la composition. De préférence, la teneur en polymère filmogène représente avantageusement de 5 à 90 % en poids par rapport au poids de la composition.

La composition selon l'invention peut comprendre en outre au moins un agent plastifiant.

Les agents plastifiants, s'ils sont présents, sont choisis parmi les composés utilisés classiquement dans ce domaine.

Plus particulièrement, l'agent plastifiant est choisi parmi la glycérine, le sorbitol, les mono et/ou disaccharides, le dipropylène glycol, le butylène glycol, le pentylène glycol ou polyéthylène glycol, tel que par exemple le PEG-400, le PEG-4000.

S'ils sont présents dans la composition, la teneur en agent plastifiant représente de préférence de 0,05 à 20 % en poids par rapport au poids de la composition.

La composition selon l'invention peut en outre comprendre au moins un agent oxydant ou au moins un agent réducteur.

En ce qui concerne l'agent oxydant, celui-ci est plus particulièrement choisi parmi le peroxyde d'hydrogène ; le peroxyde d'urée ; les percarbonates, perborates, periodates de métaux alcalins, alcalino-terreux ou d'ammonium ; les persulfates de métaux alcalins, alcalino-terreux ou d'ammonium ; les bromates de métaux alcalins, alcalino-terreux ou d'ammonium ; les ferricyanures, les sels de cuivre ou de manganèse, les quinones oxydantes, seuls ou en mélanges.

De préférence l'agent oxydant est choisi parmi le peroxyde d'hydrogène ou les persels.

En ce qui concerne l'agent réducteur, celui-ci est avantageusement choisi parmi les réductones ou les composés comprenant du soufre, et notamment des composés présentant au moins une fonction thiol, sulfite, sulfinique, sous forme de sel ou non.

Parmi les réductones, on peut citer entre autres l'acide (iso)ascorbique, l'acide érythorbique, sous forme acide, estérifiée ou encore salifiée. De manière particulièrement avantageuse conviennent l'acide ascorbique, l'acide isoascorbique, sous forme acide ou de l'un de ses sels comme le sel de sodium.

Parmi les thiols utilisables en tant que composé réducteur, on peut citer l'acide thioglycolique, le β-mercaptoéthanol, l'acide thiolactique, leurs sels de métal alcalin ou alcalino-terreux (comme le sodium, le potassium, le calcium) et leurs esters ; la cystéine, la cystéamine et leurs dérivés ; l'homocystéine et l'un de ses sels ; le mercaptoaldéhyde ; la pénicillamine ; le glutathione ; le thioglycolate de glycérol. Ces composés pouvant être utilisés seuls ou en mélanges.

En ce qui concerne les sulfites utilisables en tant que réducteurs, parmi lesquels figurent aussi les bisulfites, les hydrosulfites, conviennent les sels de métaux alcalins, alcalino-terreux ou d'ammonium, ainsi que leurs mélanges. Plus particulièrement, on peut citer le sulfite de sodium et l'hydrosulfite de sodium.

En ce qui concerne les sulfures, parmi lesquels figurent aussi les disulfures, on peut citer notamment la cystine.

Pour ce qui a trait aux composés sulfiniques, on peut citer l'hydroxyméthane sulfinate de sodium et les composés connus, décrits et préparés dans la demande de brevet WO 99/18067 et WO02/30369.

On pourra aussi mettre en oeuvre à titre d'agent réducteur, les dérivés d'acide sulfinique notamment décrits dans WO03/026597 et WO03/041668.

De préférence, l'agent réducteur, s'il est présent, est choisi parmi l'acide ascorbique, l'hydroxyméthane sulfinate de sodium, les thiols, le sulfite de sodium, seuls ou en mélanges.

La teneur en agent oxydant dans la composition, s'il est présent, représente de 0,1 à 40 % en poids, par rapport au poids de la composition.

La teneur en agent réducteur dans la composition, s'il est présent, représente de 0,1 à 20 % en poids, par rapport au poids de la composition.

La composition selon l'invention peut aussi comprendre au moins un agent régulateur de pH, alcalin ou acide.

Parmi les agents alcalinins on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 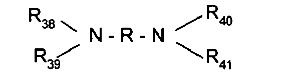 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Pour ce qui a trait aux agents acides, on peut citer par exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques, ou leurs mélanges.

La quantité d'agent alcalin et/ou acide est telle que le pH de la composition, mise en contact avec de l'eau, soit compris entre 1 et 11.

La composition selon l'invention peut aussi comprendre les additifs classiques rencontrés dans des formulations destinées à la coloration de fibres kératiniques, dès l'instant que la composition selon l'invention conserve l'aspect d'un film et que sa capacité à se désagréger en présence d'un milieu aqueux ne soit pas altérée.

A titre d'exemples, on peut citer les agents tensioactifs non ioniques, anioniques, cationiques ou amphotères ; les agents de pénétration ; les agents séquestrants ; les parfums ; les tampons ; les agents dispersants ; les agents de conditionnement tels que par exemple des cations, des polymères cationiques, des silicones volatiles ou non volatiles, modifiées ou non modifiées ; les céramides ; les agents conservateurs ; les agents stabilisants ; les agents opacifiants, etc.

Leur teneur est avantageusement comprise entre 0 et 30 % en poids par rapport au poids de la composition selon l'invention.

Selon un mode de réalisation particulier de l'invention, la composition se présente sous la forme d'un film dont l'épaisseur est comprise entre 10 et 2000 µm, plus particulièrement entre 20 et 500 µm.

Conformément à une variante de l'invention, la composition est déposée sur un support non hydrosoluble inerte vis-à-vis de la composition, et vis-à-vis de la composition lorsque celle-ci est mise en présence d'un milieu aqueux.

Avantageusement, le support non hydrosoluble est choisi parmi les polyuréthanes, les élastomères thermoplastiques du type styrène-butadiène styrène, styrène-éthylène-butadiène-styrène, éthylène vinyl acétate, ou coether ester, les polyéthylènes, les polypropylènes, les silicones, les feuilles ou films métalliques, comme l'aluminium, les feuilles ou films composites comprenant du polytétrafluoroéthylène, les copolymères polyamide à blocs polyethers, le polychlorure de vinylidène, le nylon, les élastomères de type isobutylène-styrène, styrène-isoprène, les matériaux.

De tels supports sont commercialisés notamment sous les marques : BAYDUR®, DALTOFLEX®, UROFLEX®, HYPERLAST®, INSPIRE®, DESMOPAN®, ESTANE®, LASTANE®, TEXIN®, CARIFLEX®, KRATON®, SOLPRENE®, ELVAX®, ESCORENE®, OPTENE®, ARNITEL®, HYTREL® ou RITEFLEX®.

Alternativement, le support peut être sous forme d'un non tissé, notamment en cellulose, en viscose, en coton ou en fibres synthétiques.

La nature et la forme du support seront choisies de manière adéquate afin de permettre à l'utilisateur de mettre en contact le film avec la surface à traiter, d'en permettre le massage sur la surface sans risque pour cette dernière et avec un confort maximum.

L'épaisseur du support est comprise de préférence entre 0,01 mm et 2 mm, et de préférence, entre 0,02 et 0,2 mm.

La composition selon l'invention peut être obtenue en appliquant sur un support, une composition précurseur comprenant, dans un solvant approprié, un mélange comprenant au moins un colorant direct, au moins un polymère filmogène ; puis on évapore ledit solvant.

Il est à noter que les teneurs en colorant direct et en polymère filmogène dans la composition précurseur sont telles qu'une fois le solvant évaporé, les gammes de concentrations de la composition et détaillées auparavant sont vérifiées.

La composition précurseur peut aussi comprendre au moins un agent oxydant, au moins un agent régulateur de pH, dans des proportions telles que les conditions décrites pour la composition finale soient vérifiées.

La composition peut de même comprendre des additifs classiques dans le domaine, et l'on pourra se reporter à la liste indiquée plus haut. Là encore, leur teneur dans la composition précurseur est telle qu'une fois le solvant évaporé, les gammes de concentrations dans la composition et détaillées auparavant, sont vérifiées.

Comme indiqué auparavant, la composition précurseur comprend au moins un solvant. Ce dernier est choisi de telle sorte que les composés présents dans la composition précurseur y soient solubles ou dispersés.

Avantageusement, Avantageusement, la température d'ébullition du solvant est inférieure ou égale à 200°C.

A titre d'exemples de solvants utilisables, on peut citer par exemple l'eau, l'éthanol, l'acétone, l'isopropanol, l'acétate d'éthyle, le dichlorométhane, l'éther éthylique, etc... Il est à noter que dans le cas où la composition précurseur comprend à la fois un agent oxydant et un agent régulateur de pH choisi parmi les composés alcalins, alors le solvant est de préférence différent de l'eau.

La teneur en solvant est telle qu'elle est compatible avec un étalement aisé de la composition précurseur, permettant d'en contrôler l'épaisseur.

Conformément à un mode de réalisation particulier de l'invention, la teneur totale en solvant est comprise entre 10 et 95 % en poids par rapport au poids de la composition précurseur.

La composition est obtenue en mélangeant les divers composés puis en appliquant la composition précurseur ainsi obtenue sur un support approprié comme par exemple un support non rugueux et horizontal de type marbre ou banc chauffant ou non.

Précisons que de manière avantageuse, la composition est directement déposée sur le support avec lequel la composition est destinée à être utilisée, si une telle variante est choisie.

Le dépôt de la composition est réalisé de manière classique, mais de préférence avec un appareil permettant d'obtenir une épaisseur de film substantiellement uniforme.

Après le dépôt de la composition, le solvant est évaporé de manière classique, notamment dans une étuve.

Un autre objet de l'invention est constitué par un procédé de coloration de fibres kératiniques notamment humaines, dans lequel on met en contact les fibres avec la composition selon l'invention, en présence d'un milieu aqueux.

Les fibres traitées peuvent être sèches ou humides. Dans ce dernier cas, l'eau présente sur les fibres peut constituer en totalité ou en partie le milieu aqueux mentionné ci-dessus.

Le milieu aqueux outre l'eau, peut éventuellement comprendre un ou plusieurs adjuvants différents adjuvants utilisés classiquement dans le domaine de la coloration des fibres kératiniques.

Ainsi, le milieu aqueux peut comprendre au moins un agent oxydant. Cette variante est tout particulièrement mise en oeuvre dans le cas où la composition selon l'invention est dépourvue d'agent oxydant et que l'on souhaite procéder à un éclaircissement ou une décoloration des fibres, en fonction de l'agent oxydant présent.

Le milieu aqueux peut de même comprendre un agent réducteur.

Le milieu aqueux peut de même comprendre des agents régulateurs de pH comme notamment des agents alcalinisants ou acidifiants. On pourra se reporter à la description au sujet de la nature de ces agents.

Généralement leur teneurs respectives sont tells que le pH de la composition et du milieu aqueux soit compris entre 3 et 11.

Le milieu aqueux peut aussi comprendre des adjuvants du type des tensioactifs non ioniques, anioniques, cationiques ou amphotères ; des agents de pénétration ; des agents séquestrants ; des parfums ; des tampons ; des agents dispersants ; des agents de conditionnement tels que par exemple des polymères cationiques, des silicones volatiles ou non volatiles, modifiées ou non modifiées ; des céramides ; des agents conservateurs ; des agents stabilisants ; des agents opacifiants, etc.

Le milieu aqueux peut être liquide.

Précisons que la quantité de milieux aqueux est telle qu'elle permette la libération du colorant présent dans la composition, par une désagrégation du film.

Selon un premier mode de réalisation de l'invention, on applique successivement la composition sous forme de film selon l'invention et le milieu aqueux, ou bien on applique la composition selon l'invention sur les fibres mouillées, éventuellement suivi d'une application d'un milieu aqueux complémentaire. Une fois ces opérations réalisées, il est préférable de masser les fibres afin de favoriser la désagrégation du film et la répartition du colorant ainsi libéré sur l'ensemble des fibres à traiter.

Selon un deuxième mode de réalisation particulier, on effectue, préalablement à l'application sur les fibres kératiniques, sèches ou humides, un mélange de la composition selon l'invention avec le milieu aqueux.

Quelle que soit la variante retenue, on laisse poser la composition sur les fibres jusqu'à ce que la coloration désirée soit obtenue.

A titre purement indicatif, le temps de pose varie en général d'une à 60 minutes, avantageusement de 5 à 45 minutes.

La température d'application est habituellement comprise entre 15°C et 220°C, et de préférence est voisine de la température ambiante.

Une fois le temps de pose écoulé, les fibres sont rincées, éventuellement lavées avec un shampooing puis rincées à nouveau avant d'être séchées ou laissées sécher.

Les exemples qui suivent illustrent la présente invention sans toutefois en limiter la portée.

### Exemple 1

| | |
|---|---|
| Hydroxypropyl méthylcellulose | 10 g |
| Basic Red 51 | 15 g |
| Eau | 42,5 g |

L'ensemble des ingrédients ci-dessus est mélangé sous agitation.

La solution est déposée sur un papier siliconé en une épaisseur de l'ordre de 850µm, puis séchée à une température de 50°C.

Après séchage, le film obtenu est découpé sous forme de bandelettes de 40 mm de large et de 80 mm de longueur.

On répartit 100 g d'eau sur une tête puis on applique immédiatement après deux bandelettes obtenues précédemment que l'on répartit sur la chevelure par massage.

On laisse poser 30 minutes.

Après rinçage, shampooing et séchage, la chevelure possède un reflet rouge intense.

### Exemple 2

| | |
|---|---|
| Hydroxypropyl cellulose | 10 g |
| Basic Red 51 | 5 g |
| Persulfate de potassium | 25 g |
| Ethanol | 32 g |

L'ensemble des ingrédients est mélangé sous agitation.

La dispersion est déposée sur un papier siliconé en une épaisseur de l'ordre de 1 mm, puis séchée à température ambiante.

Après séchage, le film obtenu est découpé sous forme de bandelettes de 40 mm de large et 80 mm de longueur.

On applique sur une mèche de cheveux châtain foncé 2 g d'une crème décolorante obtenue par mélange de 0,8 g d'une composition anhydre A décrite ci-dessous avec 1,2 g d'une composition aqueuse de peroxyde d'hydrogène à 6% en poids.

### Composition A

| | |
|---|---|
| Disilicate de sodium | 15 g |
| Métasilicate de sodium | 3 g |
| Chlorure d'ammonium | 4,2 g |
| EDTA | 0,2g |
| Serad FX 1100 | 2 g |
| Primogel | 2 g |
| Lauryl sulfate de sodium | 4 g |
| Stéarate de sodium | 2 g |
| Stéarate de magnésium | 2 g |
| Silice amorphe | 1 g |
| Oxyde de titane | 1 g |
| Huile minérale | 1 g |
| Cire d'abeille | 1 g |
| Myristate d'isopropyle | 21,6 g |

Immédiatement après l'application du mélange résultant, on applique sur la mèche deux bandelettes obtenues précédemment que l'on répartit sur la mèche par massage.

On laisse poser 40 minutes.

Après rinçage et séchage, on obtient une mèche blonde à reflets auburn.

## Revendications

1. Composition anhydre sous forme de film comprenant au moins un polymère filmogène et au moins un colorant direct, la teneur totale en colorant direct étant d'au moins 3 % en poids par rapport au poids de la composition.

2. Composition selon la revendication précédente, **caractérisée en ce que** le colorant direct est choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

3. Composition selon la revendication précédente, **caractérisée en ce que** la teneur totale en colorant direct est comprise entre 3 et 99,5 % en poids par rapport au poids de la composition.

4. Composition selon la revendication précédente, **caractérisée en ce que** la teneur totale en colorant direct est comprise entre 10 et 80 % en poids par rapport au poids de la composition.

5. Composition selon l'une quelconque des revendications précédentes, **caractérisée en ce que** le polymère filmogène est choisi parmi les Composition selon l'une quelconque des revendications précédentes, **caractérisée en ce que** le polymère est filmogène est hydrosoluble ou hydrodispersable.

6. Composition selon la revendication précédente, **caractérisée en ce que** le polymère est filmogène est hydrosoluble.

7. Composition selon l'une quelconque des revendications précédentes, **caractérisée en ce que** la teneur en polymère filmogène représente de 0,5 à 97 % en poids par rapport au poids de la composition.

8. Composition selon l'une quelconque des revendications précédentes, **caractérisée en ce qu'**elle comprend au moins un agent plastifiant.

9. Composition selon la revendication précédente, **caractérisée en ce que** l'agent plastifiant est choisi parmi la glycérine, le sorbitol, les mono et/ou disaccharides, le dipropylène glycol, le butylène glycol, le pentylène glycol ou polyéthylène glycol.

10. Composition selon l'une quelconque des revendications 8 ou 9, **caractérisée en ce que** la teneur en agent plastifiant représente de 0,05 à 20 % en poids par rapport au poids de la composition.

11. Composition selon l'une quelconque des revendications précédentes, **caractérisée en ce que** le film présente une épaisseur comprise entre 10 et 2000 µm, plus particulièrement entre 20 et 500 µm.

12. Composition selon l'une quelconque des revendications précédentes, **caractérisée en ce que** la composition comprend une teneur en eau est inférieure à 10 % en poids, plus particulièrement inférieure à 5 % en poids, de préférence inférieure à 3 % en poids, par rapport au poids de la composition.

13. Composition selon l'une quelconque des revendications précédentes **caractérisée en ce qu'**elle comprend au moins un agent oxydant.

14. Composition selon la revendication précédente, **caractérisée en ce que** l'agent oxydant est choisi parmi le peroxyde d'hydrogène ; le peroxyde d'urée ; les percarbonates, perborates, periodates de métaux alcalins, alcalino-terreux ou d'ammonium ; les persulfates de métaux alcalins, alcalino-terreux ou d'ammonium ; les bromates de métaux alcalins, alcalino-terreux ou d'ammonium ; les ferricyanures, les sels de cuivre ou de manganèse, les quinones oxydantes, seuls ou en mélanges.

15. Composition selon l'une quelconque des revendications 1 à 12, **caractérisée en ce que** la composition comprend au moins un agent réducteur.

16. Composition selon la revendication précédente, **caractérisée en ce que** l'agent réducteur est choisi parmi les réductones ou les composés comprenant du soufre, et notamment des composés présentant au moins une fonction thiol, sulfite, sulfinique, sous forme de sel ou non.

17. Composition selon l'une quelconque des revendications précédentes, **caractérisée en ce que** la composition comprend au moins un agent régulateur de pH.

18. Composition selon l'une quelconque des revendications précédentes, **caractérisée en ce qu'**elle est déposée sur un support non hydrosoluble.

19. Composition selon la revendication précédente, **caractérisée en ce que** le support non hydrosoluble est choisi parmi les polyuréthanes, les élastomères thermoplastiques du type styrène-butadiène styrène, styrène-éthylène-butadiène-styrène, éthylène vinyl acétate, ou coether ester, les polyéthylènes, les polypropylènes, les silicones, les feuilles ou films métalliques, comme l'aluminium, les feuilles ou films composites comprenant du polytétrafluoroéthylène, les copolymères polyamide à blocs polyethers, le polychlorure de vinylidène, le nylon, les élastomères de type isobutylène-styrène, styrène-isoprène ; les matériaux non tissés notamment en cellulose, en viscose, en coton ou en fibres synthétiques.

20. Procédé de préparation d'une composition selon l'une quelconque des revendications 1 à 17, **caractérisé en ce que** l'on applique sur un support, une composition précurseur comprenant, dans un solvant approprié, un mélange comprenant au moins un colorant direct, au moins un polymère filmogène ; puis on évapore ledit solvant.

21. Procédé selon la revendication 20, **caractérisé en ce que** la composition précurseur comprend une teneur totale en solvant comprise entre 10 à 95 % en poids par rapport au poids de la composition liquide.

22. Procédé de coloration de fibres kératiniques, dans lequel on met en contact lesdites fibres et la composition selon l'une quelconque des revendications 1 à 19, en présence d'un milieu aqueux.

23. Procédé selon la revendication précédente, **caractérisé en ce que** le milieu aqueux comprend au moins un agent oxydant.

24. Procédé selon la revendication 22, **caractérisé en ce que** le milieu aqueux comprend au moins un agent réducteur.

25. Procédé selon l'une des revendications 20 à 24, **caractérisé en ce que** l'on applique successivement la composition et le milieu aqueux.

26. Procédé selon l'une des revendications 20 à 24, **caractérisé en ce que** l'on applique le mélange extemporané de la composition et du milieu aqueux.
